# EUROPEAN PATENT SPECIFICATION

(11) **EP 3 534 258 B1**
(45) Date of publication and mention of the grant of the patent: **26.05.2021**
(21) Application number: 18159523.2
(22) Date of filing: 01.03.2018
(51) Int. Cl.: G06F 11/07, G06F 11/22, G06F 11/277

(54) **METHOD OF PERFORMING FAULT MANAGEMENT IN AN ELECTRONIC APPARATUS**
VERFAHREN ZUR FEHLERVERWALTUNG BEI EINER ELEKTRONISCHEN VORRICHTUNG
PROCÉDÉ PERMETTANT D'EFFECTUER UNE GESTION DES PANNES DANS UN APPAREIL ÉLECTRONIQUE

(43) Date of publication of application: 04.09.2019
(73) Proprietor: Siemens Healthcare GmbH, 91052 Erlangen (DE)
(72) Inventor: Hegendörfer, Friedrich, 91365 Weilersbach (DE); Manchinu, Marco, 91080 Spardorf (DE); de Oliveira, Andre, 91080 Uttenreuth (DE)

(56) References cited:
- US-A1- 2003 140 141
- US-A1- 2009 125 757
- US-A1- 2015 317 337
- US-B1- 6 325 540

## Description

Method of performing fault management in an electronic medical imaging apparatus. The invention describes a method of performing fault management in an electronic medical imaging apparatus and a fault management system for electronic medical imaging apparatus. An electronic medical imaging apparatus can be any device that comprises components for performing one or more specific functions (e.g. imaging) as well as processors and controllers to control the components. An electronic medical imaging apparatus may be a medical device, such as an MRI (magnetic resonance imaging) scanner, a CT (computed tomograph) scanner, a PET (positron emission tomography) scanner, an X-Ray apparatus, etc. These types of electronic apparatus are generally very expensive machines and must be available essentially continuously in order to be cost-effective. The reliable performance of such electronic apparatus can be helped to some extent by regular maintenance and by ensuring that the customer or user of the machine has received appropriate training. However, faults or defects will eventually occur even when such measures are adhered to. To minimize downtime of such electronic apparatus, any fault or defect should be identified and remedied as quickly as possible.

Generally, the first indication of a fault or problem is an error code or warning message shown in a display of the electronic apparatus, for example in the display of an MRI scanner. The usual way of dealing with such a reported fault is for the user - medical personnel and/or a service technician - to first gather information about the fault, for example to note the warning or error code shown in the display. A next step can be to consult a manual to determine the cause of the defect, and to carry out any remedial steps associated with that warning or error code. However, a warning or an error code may be associated with various possible causes, so that the user may need to rely on experience in an attempt to identify the precise cause of the defect. A service technician may connect a portable computer such as a tablet to the electronic apparatus in order to run a diagnostic program that may be able to identify the problem. However, it is often not possible to clearly identify the cause of the problem, and various possible solutions must be tried in an effort to localize a fault. For example, in an attempt to solve a problem in an MRI scanner, the user may replace one of the coils and then run a test sequence to see whether or not the problem has been fixed.

Clearly, such an approach involves guesswork and may be quite effective, resulting in significant and unnecessary costs by replacing parts that were not even defective. Furthermore, the time spent looking for the fault can involve machine downtime and/or service technician effort, both of which can result in significant costs. US2009/0125757A1 proposes a method in which warnings or error codes are analyzed by a monitoring system which then suggests an appropriate diagnostic workflow that can be carried out by a user in a trouble-shooting procedure. However, this approach is limited to locating problems that have developed to a stage at which warnings or error codes are generated, and cannot be used to identify a problem or defect at an earlier stage of development in an electronic apparatus.

It is therefore an object of the invention to provide a more efficient and cost-effective way of dealing with defects in such electronic medical imaging apparatus.

This object is achieved by the method of claim 1 of performing fault management in electronic medical imaging apparatus; by the fault management system of claim 12; the computer program product according to claim 13; and the computer readable medium according to claim 14.

According to the invention, the method of performing fault management in electronic medical imaging apparatus (referred to in the following simply as "electronic apparatus") comprises the steps of transferring machine data of the electronic apparatus to a remote support centre (referred to in the following as the "backend"), wherein machine data comprises information generated by electronic apparatus in the course of normal operation and comprises any of: machine parameters for an imaging task, statements summarizing the steps of an imaging procedure, sensor output values, parameters set by a user, parameters set by the device, images generated during an imaging task, etc. The method further comprises the step of applying artificial intelligence in the form of a neural network to analyse the machine data of the electronic apparatus in the backend, which neural network has learned to identify expected content in machine data and to recognize any departure from such expected content. The method further comprises the steps of providing a diagnostic/corrective workflow in response to an anomaly detected in machine data; and operating the electronic apparatus from the backend to execute the diagnostic or corrective workflow, wherein machine data of the diagnostic and/or corrective workflow is analysed in the remote support centre in real time.

The term "backend" has its origin in information systems, and generally refers to a database management system (DBMS) which can store and analyse large quantities of data. A database management system may also be assumed to include software such as a web server or application server to perform data processing.

The inventive fault management method is to be understood as being autonomous in the sense that the steps of analysing the machine data, providing a diagnostic/corrective workflow and remotely operating the electronic apparatus do not require any user participation or intervention. In the context of the invention, a "workflow" is to be understood as a set of instructions for an electronic apparatus. This instruction set can cause the apparatus to perform a defined sequence of tasks, for example a workflow can make an apparatus start up, actuate one or more components to perform one or more tasks, record information resulting from the task, etc. In the case of a medical device such as an MRI scanner, a workflow can cause the scanner to start up and then to deploy various components such as the coils to perform test sequences, to record sensor readings during these test sequences, and subsequently to shut the scanner down again.

In the inventive method, machine data from an electronic apparatus are transferred to the backend during normal operation of the electronic apparatus, i.e. in real-time. As a result, the inventive method can continually collect normal operating information from any electronic apparatus being supported by that backend. Analysis of the machine data can therefore continually run in the background without any need for a user of the electronic apparatus to initiate a diagnostic session.

An advantage of the inventive method is that it overcomes the limitations of the prior art approach of running test routines which are generally only run after a problem has been reported by an error code or warning message. As explained above, the prior art usually involves some guesswork, for example running test routines in an attempt to identify the source of a problem and/or replacing parts in the attempt to localise a problem even if there is no clear indication that the exchanged part is faulty.

In contrast, the approach taken by the inventive method is to apply artificial intelligence at an interface between the electronic apparatus and the backend, and to analyse the machine data. The term "artificial intelligence" is to be understood in its established context, i.e. to include any suitable machine learning algorithm such as a neural network. Machine data of an electronic apparatus is to be understood as any information generated by the electronic apparatus in the course of normal operation, for example in the course of an imaging task. For example, machine data of an MR device can comprise the machine parameters for that imaging task, a series of statements summarizing the steps of the imaging procedure, sensor output values, etc. The machine parameters can be parameters that were set by the user and/or parameters that are set by the device itself. Such machine data may also include one or more images generated during the imaging task. In the inventive method, machine data are analysed and compared to expected content so that any departure from the expected performance can be immediately identified and evaluated. If such a discrepancy is detected and considered significant, the backend identifies one or more diagnostic/corrective workflows (or "service software workflows") that will likely locate the cause of the unexpected behaviour. A workflow can serve to diagnose a problem (diagnostic workflow) and/or to correct or remedy the problem (corrective workflow). In the following, the term diagnostic workflow may be assumed to also comprise corrective workflow elements even if not explicitly stated. The electronic apparatus is operated from the backend to execute the diagnostic workflow. In other words, the backend controls the electronic apparatus to carry out the diagnostic workflow. Any machine data generated during the diagnostic workflow is transferred to the backend so that the outcome of the diagnostic workflow can be immediately evaluated. It may be that the problem can be dealt with remotely by altering a device parameter or device setting from the remote service centre. Equally, if a fault can be identified and the problem can be dealt with by the medical personnel on site, the remote service centre can provide a set of instructions (for example to exchange a defective part). If the problem can only be dealt with by a service technician, this can be scheduled. If the problem was not identified in the diagnostic workflow, an alternative workflow can be chosen and the process can be repeated. The inventive approach can therefore lead to significant savings by avoiding unnecessary replacement of non-defective parts. The number of service personnel site visits can also be reduced so that savings may be made here also.

The inventive method also makes it possible to identify system-specific corrective actions which can be performed even before a problem has actually become noticeable. The inventive method can therefore maximise system uptime and can avoid or reduce service contract uptime violations.

According to the invention, the fault management system comprises a means of transferring machine data of electronic apparatus to a backend during normal operation of the electronic apparatus, wherein machine data comprises information generated by electronic apparatus in the course of normal operation and comprises any of: machine parameters for an imaging task, statements summarizing the steps of an imaging procedure, sensor output values, parameters set by a user, parameters set by the device, images generated during an imaging task. The fault management system further comprises an analysis arrangement realised to apply artificial intelligence in the form of a neural network to analyse the machine data of the electronic apparatus in the backend, which neural network learns to identify expected content in machine data and to recognize any departure from such expected content; and to identify a diagnostic and/or corrective workflow for an electronic apparatus in response to an anomaly detected in machine data of that electronic apparatus, and to analyse the machine data of the diagnostic and/or corrective workflow in the remote support centre in real time. The fault management system is realised to allow operation of the electronic medical imaging apparatus from the remote support centre to carry out the diagnostic and/or corrective workflow.

The object of the invention is also achieved by a computer program product that is capable of carrying out the steps of the inventive method when loaded into a programmable device of the electronic apparatus fault management system. The computer program product can comprise a computer program that is directly loadable into the memory of a control unit of the electronic apparatus fault management system, and which comprises program units to perform the steps of the inventive method when the program is executed by the control unit.

Particularly advantageous embodiments and features of the invention are given by the dependent claims, as revealed in the following description. Features of different claim categories may be combined as appropriate to give further embodiments not described herein.

In a particularly preferred embodiment of the invention, the electronic apparatus comprises one or more medical imaging devices such as an MRI scanner, a CT scanner, a PET scanner, an X-Ray apparatus, etc.

In the following, but without restricting the invention in any way, it may be assumed that an electronic apparatus comprises a medical device such as an MRI scanner or a CT scanner, so that the terms "electronic apparatus" and "medical device" may be used interchangeably. Such medical devices are usually designed to record every action or event, as well as any machine settings or parameters. Information entered by a user may also be recorded. Information generated by the machine during a task or workflow is generally also recorded. Collectively, this information is referred to as the "machine data" or "performance log", and these terms may also be used interchangeably in the following.

In the following, it may be assumed that the remote support centre or backend is capable of providing support for any number of medical devices that may be located at one or more customer sites such as hospitals, clinics, research facilities, etc.

When a medical device such as an MRI scanner or a CT scanner is set up to perform an imaging task for a patient, information pertaining to the patient is generally entered into the machine and stored with the image data that is generated during the imaging task. Since this information is then part of the performance log, steps are preferably taken to anonymise the patient data before transmitting the data to the remote service centre. Furthermore, this information comprises sensitive data that should be protected and prevented from access by third parties. Therefore, without restricting the invention in any way, it may be assumed in the following that communication between a medical device and the remove service centre is carried out over a secure connection, for example using a VPN (virtual private network) to enable information to be exchanged between the customer site and the remote support centre or backend using a public network such as the internet. Preferably, the medical device fault management system is realised to allow operation of the medical device from the backend, i.e. to execute a diagnostic workflow without any interaction from a user at the medical device location.

In the inventive method, analysis of real-time machine data can reliably identify an existing problem or even a potential problem, i.e. a problem that has not yet developed to the point where the medical device would issue a warning or error message. The inventive method applies artificial intelligence to identify a diagnostic workflow, i.e. a system-specific automated workflow. The diagnostic workflow is uploaded from the backend directly to the medical device and executed on the electronic apparatus. The interface between the electronic apparatus and the remote service centre carries out the machine data analysis and diagnostic workflow selection autonomously and without any user interaction. The "interface" between the medical device and the remote service centre may therefore be understood as an "artificial intelligence interface" or "AI interface", since it can learn from machine data to deduce diagnostic information and can autonomously apply this information by initiating remote execution of a suitable diagnostic workflow. For example, when a system-specific automated workflow is run on a medical device such as a Magnet Resonance Imaging System, the performance data collected during the workflow may indicate an unexpected result. In the inventive method, a diagnostic workflow that will likely pinpoint this problem is created, or chosen from a set of already existing workflows, and executed remotely from the service centre. The machine data of the diagnostic workflow is analysed in real time to determine whether or not the problem was localized. The AI interface furthermore will try to home in on the root cause of the problem by identifying one or more specific part(s) that might possibly be defective and which might need to be replaced. In the above example, a dedicated diagnostic workflow explicitly chosen or created by the AI interface is sent to the Magnet Resonance Imaging system. The diagnostic workflow runs on the MRI system to collect information about potentially defective parts. The machine data collected during the diagnostic workflow is evaluated. The outcome may be that some of the potentially defective parts turn out to be alright, and the problem can be pinpointed in a single part. A suggested remedy may be to replace that part if the AI evaluation indicates that the part is truly defective. Alternatively, the AI evaluation might indicate that an adjustment procedure (tuning) might solve the problem. In this way, failure of a part (e.g. a coil, a transmitter, a receiver, etc.) can be avoided by running an adjustment procedure in good time, thereby avoiding unnecessary part replacement. Either way, costly downtime is avoided.

An artificial intelligence arrangement can implement any suitable machine learning method, for example a random tree. According to the invention, the artificial intelligence interface implements a method that comprises an artificial neural network. This can be realised in any suitable manner. For example, the artificial neural network can be realised as a deep neural network or a deep belief network. The artificial neural network can simply be fed with machine data as they are generated by any medical device supported by the remote service centre. As indicated above, machine data can comprise parameter values of the medical device, for example input settings, voltage and current curves, sensor output values, etc. A specific imaging task with a certain set of input parameters should result in a corresponding performance log. The step of analysing machine data preferably comprises detecting any deviation from an expected pattern in the performance log. The artificial neural network learns to identify expected content in machine data and to recognise any departure from such expected content. This learning can be done in a supervised manner initially if required; alternatively the artificial neural network may be allowed to learn in an entirely unsupervised manner. The artificial neural network can learn from a database of machine data collected in the past, for example. A favourably high accuracy of the artificial neural network at the outset can be achieved by providing a sufficiently large database from which the artificial neural network can learn.

By continually learning from collected machine data over weeks, months or even years of use under various conditions, the inventive method can continually refine, extend and improve a set of diagnostic workflows for medical devices such as MRI scanners, CT scanners, or any electronic apparatus that generates comprehensive machine data. An advantage of the inventive method is that optimization of diagnostic workflows is taken care of in the backend. Of course, improvements arising from analysis of the machine data can be used to benefit electronic apparatus by installing appropriate software updates at a convenient time, for example during a scheduled maintenance procedure. For example, the intelligent interface may have learned that development of a certain fault is associated with a specific pattern of reports from a particular sensor, and a software update can be prepared that generates a suitable warning message to alert the user of the electronic apparatus in good time.

Machine data from an imaging device such as an MRI scanner may also include the (anonymised) image data. Here, the step of analysing the machine data preferably also comprises a step of assessing the quality of the image data. Image quality must be high in order for a correct diagnosis to be made for that patient. However, the quality of the images generated by such a medical device depends on many factors, for example signal-to-noise ratio, stability of the base field, gradient gain, etc. Even for trained medical personnel, it can be difficult and time-consuming to identify the reason for poor image quality. In the inventive method, it is relatively easy for the artificial intelligence interface to provide a diagnostic workflow that can find the cause of a decrease in image quality, and to suggest remedial action in order to improve image quality in subsequent imaging tasks. This can favourably reduce or eliminate the need to repeat expensive imaging procedures.

As indicated above, a diagnostic workflow for an electronic apparatus is preferably created and/or optimized on the basis of knowledge obtained from the analysis of multiple instances or sets of machine data. The knowledge obtained from the analysis of multiple instances or sets of machine data can comprise any of event history (service activity data) for that electronic apparatus type; a defect history (compendium of all defects including solution results as available from previous activities) for that electronic apparatus type; spare part consumption history (parts in / parts out) for that electronic apparatus type, etc. Such a collection of information may be regarded as "big data" from which suitable workflows may be created for the electronic apparatus.

Any diagnostic workflow is preferably uploaded from the backend to the electronic apparatus, and the electronic apparatus is operated remotely from the backend to execute the diagnostic workflow. Of course, if necessary or if preferred, the electronic apparatus can be operated locally by a service technician in order to execute an uploaded diagnostic workflow.

Various units or modules of the fault management system mentioned above can be completely or partially realised as software modules running for example on a processor of a suitable control unit of the fault management system, for example a control unit of the analysis arrangement. A realisation largely in the form of software modules can have the advantage that applications already operational in an existing remote service centre system can be updated, with relatively little effort, to install and run the fault management method of the present application.

A computer readable medium such as a memory stick, a hard-disk or other transportable or permanently-installed carrier can serve to transport and/or to store the executable parts of the computer program product so that these can be read by a processor unit of the fault management system. A processor unit can comprise one or more microprocessors or their equivalents.

Other objects and features of the present invention will become apparent from the following detailed descriptions considered in conjunction with the accompanying drawings. It is to be understood, however, that the drawings are designed solely for the purposes of illustration and not as a definition of the limits of the invention.
Fig 1 shows an embodiment of the inventive fault management system;
Fig 2 shows a block diagram of an analysis arrangement in an embodiment of the inventive fault management system;
Fig 3 shows a prior art environment including medical devices and a remote service centre.

In the diagrams, like numbers refer to like objects throughout. Objects in the diagrams are not necessarily drawn to scale.

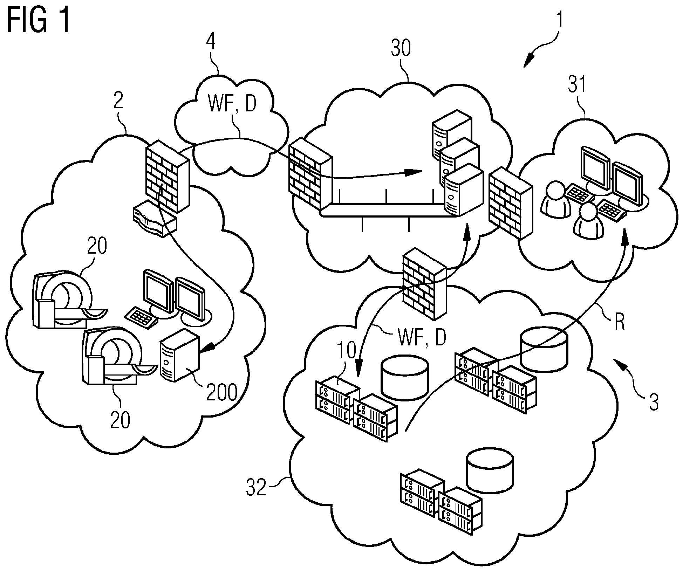

Fig 1 shows an embodiment of the inventive fault management system 1, giving a simplified overview of the relationship between the various elements. Here, a customer local area network (LAN) 2 is shown to include electronic apparatus 20 in the form of two exemplary medical devices 20, in this case imaging devices 20. Of course, the customer LAN - which may be a hospital LAN - may include any number of medical devices. A medical device 20 is operated by means of a computer interface 200 indicated on the right. A user or device operator may set up the imaging device 20 for a specific task with the user interface (keyboard, monitor, etc.). The medical device 20 generates machine data D during operation, and this is sent (usually in encrypted form) over a wide-area network (WAN) internet to a perimeter network 30 or "demilitarized zone" 30 of a remote service centre (RSC) 3. Protective firewall measures are indicated by the "wall" symbols. The RSC 3 can support any number of customers, but for the sake of clarity only one customer is indicated here by the customer LAN 2. The RSC 3 further comprises an intranet 31 that allows service centre personnel to monitor any devices supported by that remote service centre. Data storage and data analytics are outsourced to a data management zone 32 for managing storage of large volumes of data and for performing computationally intensive analytical tasks.

From the demilitarized zone 30, the machine data D are passed directly to an analysis arrangement 10 in the data management zone 32 or DBMS 32. The analysis arrangement 10 applies artificial intelligence in the form of a neural network such as a deep learning network to analyse machine data D and to learn from machine data D to detect any discrepancy from expected content. If machine data D is considered to be "normal" i.e. to have a "no fault detected" status, the artificial intelligence module 10 will conclude that no remedial action is necessary. However, if an anomaly is detected, the artificial intelligence module 10 will choose a diagnostic workflow WF from a collection of existing diagnostic workflows, or will create a suitable diagnostic workflow WF. The workflow will be uploaded to the customer LAN 2 over the same communications channel (e.g. a secure VPN tunnel) and executed on the relevant electronic apparatus 20. Depending on the outcome of the diagnostic workflow WF, personnel at the RSC 3 may be informed and/or the user at the customer LAN 2 may be instructed to carry out remedial actions.

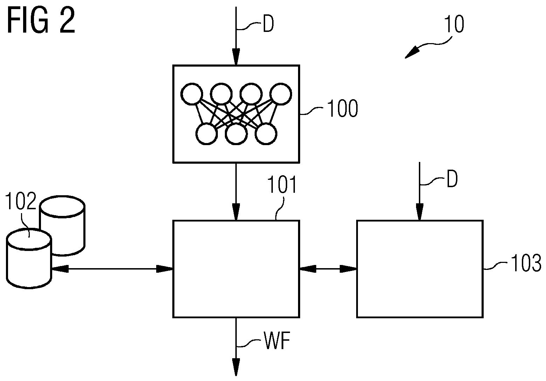

Fig 2 shows a block diagram of an exemplary analysis arrangement 10. One or more sets of machine data D are received by an artificial intelligence module 100 such as a deep neural network 100. While only a single set or instance of machine data D may be received at any one time from an electronic apparatus, multiple sets or instances of machine data D may be received over time, and these all contribute to the learning development of the deep neural network 100. This analyses machine data D with the aim of detecting any anomaly or departure from an expected content. If the machine data D indicates that the medical device is not operating satisfactorily, a module 101 of the analysis arrangement 10 identifies a suitable diagnostic/corrective workflow WF, either by choosing it from an existing workflow collection 102 or by running a workflow generator 103 to create a new diagnostic/corrective workflow using device-relevant information from the machine data D. As indicated in Fig 1, the workflow WF is uploaded to the medical device 20 and executed. This step is also performed remotely, i.e. from the RSC 3 and without any interaction required on the part of the customer (medically trained personal on site 2) or the service centre personnel.

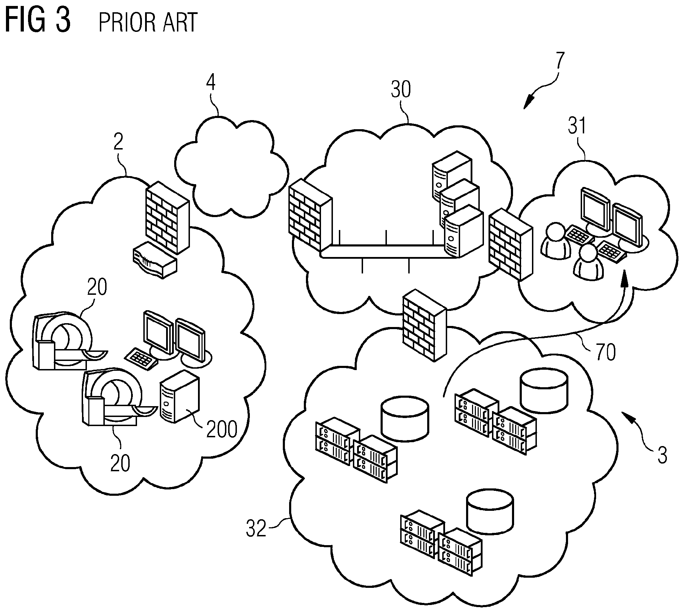

Fig 3 shows a prior art environment 7 with a customer local area network (LAN) 2 and a remote service centre 3. The configuration is similar to that of Fig 1, with medical devices 20 of a customer LAN 2 connected via a WAN 4 to an RSC 3, and with data storage and data analytics outsourced to an external provider 32. However, in the prior art, a complex fault in an electronic apparatus may have multiple potential root causes so that it can only be identified by trial and error for example by replacing parts and then checking to see if the problem has been fixed. Alternatively, if a problem is identified by RSC personnel, these may notify the customer by updating relevant web application pages or by telephone/e-mail contact, for example.

Although the present invention has been disclosed in the form of preferred embodiments and variations thereon, it will be understood that numerous additional modifications and variations could be made thereto without departing from the scope of the invention.

For the sake of clarity, it is to be understood that the use of "a" or "an" throughout this application does not exclude a plurality, and "comprising" does not exclude other steps or elements. The mention of a "unit" or a "module" does not preclude the use of more than one unit or module.

## Claims

1. A method of performing fault management in electronic medical imaging apparatus (2), which method comprises the steps of
- transferring machine data (D) of electronic medical imaging apparatus (2) to a remote support centre (3) during normal operation of the electronic medical imaging apparatus (2), wherein machine data (D) comprises information generated by electronic medical imaging apparatus (2) in the course of normal operation and comprises any of: machine parameters for an imaging task, statements summarizing the steps of an imaging procedure, sensor output values, parameters set by a user, parameters set by the device, images generated during an imaging task;
- applying artificial intelligence in the form of a neural network (100) to analyse machine data (D) of electronic medical imaging apparatus (2) in the remote support centre (3), wherein the neural network (100) has learned to identify expected content in machine data (D) and to recognise any departure from such expected content;
- providing a diagnostic and/or corrective workflow (WF) in response to an anomaly detected in machine data (D) of an electronic medical imaging apparatus (2);
- operating that electronic medical imaging apparatus (2) from the remote support centre (3) to execute the diagnostic and/or corrective workflow (WF), wherein machine data (D) of the diagnostic and/or corrective workflow (WF) is analysed in the remote support centre (3) in real time.

2. A method according to claim 1, wherein machine data is transferred from electronic medical imaging apparatus comprising any of an MRI scanner, a CT scanner, a PET scanner, an X-Ray apparatus.

3. A method according to claim 1 or claim 2, wherein the neural network (100) comprises a deep neural network.

4. A method according to claim 3, wherein multiple sets of machine data (D) received over time contribute to the learning development of the deep neural network (100).

5. A method according to any of the preceding claims, wherein machine data (D) comprises parameter values of an electronic medical imaging apparatus (2), and the step of analysing machine data (D) comprises detecting a deviation from an expected pattern and/or learning to recognise a new pattern.

6. A method according to any of the preceding claims, wherein machine data (D) comprises image data generated by the electronic medical imaging apparatus (2), and the step of analysing the machine data (D) comprises a step of assessing the quality of the image data.

7. A method according to any of the preceding claims, comprising a step of creating a diagnostic and/or corrective workflow (WF) on the basis of knowledge obtained from the analysis of multiple machine data sets (D).

8. A method according to any of the preceding claims, comprising a step of optimizing a diagnostic and/or corrective workflow (WF) for an electronic medical imaging apparatus (2) on the basis of knowledge obtained from the analysis of multiple machine data sets (D).

9. A method according to any of the preceding claims, wherein the diagnostic and/or corrective workflow (WF) for an electronic medical imaging apparatus (2) is optimised according to any of: an event history for that electronic apparatus type; a defect history for that electronic apparatus type; spare part consumption history for that electronic apparatus type.

10. A method according to any of the preceding claims, wherein the diagnostic and/or corrective workflow (WF) is uploaded from the remote support centre (3) to the electronic medical imaging apparatus (2).

11. A method according to any of the preceding claims, wherein the electronic medical imaging apparatus (2) is operated remotely from the remote support centre (3) to execute the diagnostic or corrective workflow (WF).

12. A fault management system (1) for electronic medical imaging apparatus (2), comprising
- a means of transferring machine data (D) of electronic medical imaging apparatus (2) to a remote support centre (3) during normal operation of the electronic medical imaging apparatus (2), wherein machine data (D) comprises information generated by electronic medical imaging apparatus (2) in the course of normal operation and comprises any of: machine parameters for an imaging task, statements summarizing the steps of an imaging procedure, sensor output values, parameters set by a user, parameters set by the device, images generated during an imaging task;
- an analysis arrangement (10) realised to apply artificial intelligence in the form of a neural network (100) to analyse machine data (D) of electronic medical imaging apparatus (2) in the remote support centre (3), which neural network (100) learns to identify expected content in machine data (D) and to recognize any departure from such expected content; to provide a diagnostic and/or corrective workflow (WF) for an electronic medical imaging apparatus (2) in response to an anomaly detected in machine data (D) of that electronic medical imaging apparatus (2);
and to analyse the machine data (D) of the diagnostic and/or corrective workflow (WF) in the remote support centre (3) in real time;
and wherein the fault management system (1) is realised to allow operation of the electronic medical imaging apparatus (2) from the remote support centre (3) to carry out the diagnostic and/or corrective workflow (WF).

13. A computer program product comprising a computer program that is directly loadable into a memory of a control unit of a fault management system (1) of claim 12, and which comprises program elements for performing steps of the method according to any of claims 1 to 11 when the computer program is executed by the control unit of the fault management system (1).

14. A computer readable medium to transport and/or to store the executable parts of the computer program product of claim 13 so that these can be read by a processor unit of the fault management system (1).

## Patentansprüche

1. Verfahren zum Durchführen einer Fehlerverwaltung in einer elektronischen medizinischen Bildgebungseinrichtung (2), wobei das Verfahren die folgenden Schritte umfasst:
- Übertragen von Maschinendaten (D) einer elektronischen medizinischen Bildgebungseinrichtung (2) an ein entferntes Unterstützungszentrum (3) während des normalen Betriebs der elektronischen medizinischen Bildgebungseinrichtung (2), wobei Maschinendaten (D) Informationen umfassen, die von der elektronischen medizinischen Bildgebungseinrichtung (2) im Verlauf des normalen Betriebs erzeugt werden, und eines der Folgenden umfassen: Maschinenparameter für eine Bildgebungsaufgabe, Anweisungen, die die Schritte eines Bildgebungsverfahrens zusammenfassen, Sensorausgangswerte, von einem Benutzer eingestellte Parameter, von der Vorrichtung eingestellte Parameter, Bilder, die während einer Bildgebungsaufgabe erzeugt werden;
- Anwenden von künstlicher Intelligenz in Form eines neuronalen Netzes (100), um Maschinendaten (D) einer elektronischen medizinischen Bildgebungseinrichtung (2) im entfernten Unterstützungszentrum (3) zu analysieren, wobei das neuronale Netz (100) gelernt hat, erwartete Inhalte in Maschinendaten (D) zu identifizieren und jede Abweichung von solchen erwarteten Inhalten zu erkennen;
- Bereitstellen eines diagnostischen und/oder korrigierenden Arbeitsablaufs (Workflow, WF) in Reaktion auf eine Anomalie, die in Maschinendaten (D) einer elektronischen medizinischen Bildgebungseinrichtung (2) erkannt wurde;
- Bedienen der betreffenden elektronischen medizinischen Bildgebungseinrichtung (2) vom entfernten Unterstützungszentrum (3) aus, um den diagnostischen und/oder korrigierenden Arbeitsablauf (WF) auszuführen, wobei die Maschinendaten (D) des diagnostischen und/oder korrigierenden Arbeitsablaufs (WF) im entfernten Unterstützungszentrum (3) in Echtzeit analysiert werden.

2. Verfahren gemäß Anspruch 1, wobei Maschinendaten von einer elektronischen medizinischen Bildgebungseinrichtung übertragen werden, die eine(n) beliebige(n) MRI-Scanner, CT-Scanner, PET-Scanner oder Röntgeneinrichtung umfasst.

3. Verfahren gemäß Anspruch 1 oder Anspruch 2, wobei das neuronale Netz (100) ein tiefes neuronales Netz umfasst.

4. Verfahren gemäß Anspruch 3, wobei mehrere Sätze von Maschinendaten (D), die im Laufe der Zeit empfangen werden, zur Lernentwicklung des tiefen neuronalen Netzes (100) beitragen.

5. Verfahren gemäß einem der vorstehenden Ansprüche, wobei Maschinendaten (D) Parameterwerte einer elektronischen medizinischen Bildgebungseinrichtung (2) umfassen und der Schritt des Analysierens von Maschinendaten (D) ein Erkennen einer Abweichung von einem erwarteten Muster und/oder ein Erlernen eines Erkennens eines neuen Musters umfasst.

6. Verfahren gemäß einem der vorstehenden Ansprüche, wobei Maschinendaten (D) Bilddaten umfassen, die von der elektronischen medizinischen Bildgebungseinrichtung (2) erzeugt werden, und der Schritt des Analysierens der Maschinendaten (D) einen Schritt des Beurteilens der Qualität der Bilddaten umfasst.

7. Verfahren gemäß einem der vorstehenden Ansprüche, umfassend einen Schritt des Erstellens eines diagnostischen und/oder korrigierenden Arbeitsablaufs (WF) basierend auf Erkenntnissen, die aus der Analyse mehrerer Sätze von Maschinendaten (D) gewonnen wurden.

8. Verfahren gemäß einem der vorstehenden Ansprüche, umfassend einen Schritt des Optimierens eines diagnostischen und/oder korrigierenden Arbeitsablaufs (WF) für eine elektronische medizinische Bildgebungseinrichtung (2) basierend auf Erkenntnissen, die aus der Analyse mehrerer Sätze von Maschinendaten (D) gewonnen wurden.

9. Verfahren gemäß einem der vorstehenden Ansprüche, wobei der diagnostische und/oder korrigierende Arbeitsablauf (WF) für eine elektronische medizinische Bildgebungseinrichtung (2) gemäß einem der Folgenden optimiert wird: einer Ereignishistorie für diesen Typ von elektronischer Einrichtung; einer Fehlerhistorie für diesen Typ von elektronischer Einrichtung; einer Ersatzteilverbrauchshistorie für diesen Typ von elektronischer Einrichtung.

10. Verfahren gemäß einem der vorstehenden Ansprüche, wobei der diagnostische und/oder korrigierende Arbeitsablauf (WF) von dem entfernten Unterstützungszentrum (3) auf die elektronische medizinische Bildgebungseinrichtung (2) hochgeladen wird.

11. Verfahren gemäß einem der vorstehenden Ansprüche, wobei die elektronische medizinische Bildgebungseinrichtung (2) vom entfernten Unterstützungszentrum (3) aus bedient wird, um den diagnostischen oder korrigierenden Arbeitsablauf (WF) auszuführen.

12. Fehlerverwaltungssystem (1) für eine elektronische medizinische Bildgebungseinrichtung (2), umfassend
- ein Mittel zum Übertragen von Maschinendaten (D) einer elektronischen medizinischen Bildgebungseinrichtung (2) an ein entferntes Unterstützungszentrum (3) während des normalen Betriebs der elektronischen medizinischen Bildgebungseinrichtung (2), wobei Maschinendaten (D) Informationen umfassen, die von der elektronischen medizinischen Bildgebungseinrichtung (2) im Verlauf des normalen Betriebs erzeugt werden, und eines der Folgenden umfassen: Maschinenparameter für eine Bildgebungsaufgabe, Anweisungen, die die Schritte eines Bildgebungsverfahrens zusammenfassen, Sensorausgangswerte, von einem Benutzer eingestellte Parameter, von der Vorrichtung eingestellte Parameter, Bilder, die während einer Bildgebungsaufgabe erzeugt werden;
- eine Analyseanordnung (10), die realisiert ist zum Anwenden von künstlicher Intelligenz in Form eines neuronalen Netzes (100), um Maschinendaten (D) einer elektronischen medizinischen Bildgebungseinrichtung (2) im entfernten Unterstützungszentrum (3) zu analysieren, wobei das neuronale Netz (100) lernt, erwartete Inhalte in Maschinendaten (D) zu identifizieren und jede Abweichung von solchen erwarteten Inhalten zu erkennen; zum Bereitstellen eines diagnostischen und/oder korrigierenden Arbeitsablaufs (Workflow, WF) in Reaktion auf eine Anomalie, die in Maschinendaten (D) einer elektronischen medizinischen Bildgebungseinrichtung (2) erkannt wurde, für die elektronische medizinische Bildgebungseinrichtung (2);
und zum Analysieren der Maschinendaten (D) des diagnostischen und/oder korrigierenden Arbeitsablaufs (WF) im entfernten Unterstützungszentrum (3) in Echtzeit;
und wobei das Fehlerverwaltungssystem (1) so realisiert ist, dass es die Bedienung der elektronischen medizinischen Bildgebungseinrichtung (2) vom entfernten Unterstützungszentrum (3) aus ermöglicht, um den diagnostischen und/oder korrigierenden Arbeitsablauf (WF) auszuführen.

13. Computerprogrammprodukt, umfassend ein Computerprogramm, das direkt in einen Speicher einer Steuereinheit eines Fehlerverwaltungssystems (1) gemäß Anspruch 12 ladbar ist, und das Programmelemente zum Durchführen von Schritten des Verfahrens gemäß einem der Ansprüche 1 bis 11 umfasst, wenn das Computerprogramm von der Steuereinheit des Fehlerverwaltungssystems (1) ausgeführt wird.

14. Computerlesbares Medium zum Transportieren und/oder Speichern der ausführbaren Teile des Computerprogrammprodukts gemäß Anspruch 13, so dass diese von einer Prozessoreinheit des Fehlerverwaltungssystems (1) gelesen werden können.

## Revendications

1. Procédé pour effectuer une gestion des pannes d'une installation (2) électronique d'imagerie médicale, lequel procédé comprend les stades dans lesquels :
- on transfère une donnée (D) de machine de l'installation (2) électronique d'imagerie médicale à un centre (3) éloigné d'assistance pendant un fonctionnement normal de l'installation (2) électronique d'imagerie médicale, la donnée (D) de machine comprenant de l'information produite par l'installation (2) électronique d'imagerie médicale au cours d'un fonctionnement normal et comprenant l'un quelconque de : des paramètres de machine pour une tâche d'imagerie, des énoncés résumant les stades d'une procédure d'imagerie, des valeurs de sortie de capteur, des paramètres fixés par un utilisateur, des paramètres fixés par le dispositif, des images produites pendant une tâche d'imagerie,
- on applique l'intelligence artificielle sous la forme d'un réseau (100) neuronal, pour analyser la donnée (D) de machine de l'installation (2) électronique d'imagerie médicale dans le centre (3) éloigné d'assitance, le réseau (100) neuronal ayant appris à identifier un contenu escompté dans la donnée (D) de machine et à reconnaître tout écart à un tel contenu escompté,
- on fournit une norme (WF) de diagnostic et/ou de correction en réaction à une anomalie détectée dans la donnée (D) de machine d'une installation (2) électronique d'imagerie médicale ;
- on fait fonctionner cette installation (2) électronique d'imagerie médicale, depuis le centre (3) éloigné d'assitance, pour exécuter la norme (WF) de diagnostic et/ou de correction, la donnée (D) de machine de la norme (WF) de diagnostic et/ou de correction étant analysée en temps réel dans le centre (3) éloigné d'assistance.

2. Procédé suivant la revendication 1, dans lequel on transfert la donnée de machine depuis l'installation électronique d'imagerie médicale, comprenant l'un quelconque d'un scanner MRI, d'un scanner CT, d'un scanner PET, d'une installation à rayons X.

3. Procédé suivant la revendication 1 ou 2, dans lequel le réseau (100) neuronal comprend un réseau neuronal profond.

4. Procédé suivant la revendication 3, dans lequel de multiples ensembles de données (D) de machine, reçus en fonction du temps, contribuent au développement de l'apprentissage du réseau (100) neuronal profond.

5. Procédé suivant l'une des revendications précédentes dans lequel, la donnée (D) de machine comprend des valeurs de paramètre de l'installation (2) électronique d'imagerie médicale, et le stade d'analyse de la donnée (D) de machine comprend détecter une déviation d'une configuration escomptée et/ou apprendre à reconnaître une configuration nouvelle.

6. Procédé suivant l'une quelconque des revendications précédentes, dans lequel la donnée (D) de machine comprend une donnée d'image produite par l'installation (2) électronique d'imagerie médicale, et le stade d'analyse de la donnée (D) de machine comprend un stade d'évaluation de la qualité des données d'image.

7. Procédé suivant l'une quelconque des revendications précédentes, comprenant un stade de création d'une norme (WF) de diagnostic et/ou de correction sur la base du savoir obtenu à partir de l'analyse de multiples ensembles (D) de données de machine.

8. Procédé suivant l'une quelconque des revendications précédentes, comprenant un stade d'optimisation d'une norme (WF) de diagnostic et/ou de correction pour installation (2) électronique d'imagerie médicale sur la base du savoir obtenu à partir de l'analyse de multiples ensembles (D) de données de machine.

9. Procédé suivant l'une quelconque des revendications précédentes, dans lequel on optimise la norme (WF) de diagnostic et/ou de correction pour une installation (2) électronique d'imagerie médicale suivant l'un quelconque de : une histoire événementielle pour ce type d'installation électronique ; une histoire des pannes pour ce type d'installation électronique ; une histoire de consommation de pièces détachées pour ce type d'installation électronique.

10. Procédé suivant l'une quelconque des revendications précédentes, dans lequel on charge la norme (WF) de diagnostic et/ou de correction du centre (3) éloigné d'assistance à l'installation (2) électronique d'imagerie médicale.

11. Procédé suivant l'une quelconque des revendications précédentes, dans lequel on fait fonctionner l'installation (2) électronique d'imagerie médicale à distance depuis le centre (3) éloigné d'assistance pour exécuter la norme (WF) de diagnostic ou de correction.

12. Système (1) de gestion des pannes d'une installation (2) électronique d'imagerie médicale, comprenant
- un moyen de transfert d'une donnée (D) de machine de l'installation (2) électronique d'imagerie médicale à un centre (3) éloigné d'assistance, pendant un fonctionnement normal de l'installation (2) électronique d'imagerie médicale, la donnée (D) de machine comprenant de l'information produite par l'installation (2) électronique d'imagerie médicale au cours du fonctionnement normal et comprenant l'un quelconque de : des paramètres de machine pour une tâche d'imagerie, des énoncés résumant les stades d'une procédure d'imagerie, des valeurs de sortie de capteur, des paramètres fixés par un utilisateur, des paramètres fixés par le dispositif, des images produites pendant une tâche d'imagerie ;
- un agencement (10) d'analyse réalisé pour appliquer l'intelligence artificielle sous la forme d'un réseau (100) neuronal pour analyser la donnée (D) de machine de l'installation (2) électronique d'imagerie médicale dans le centre (3) éloigné d'assistance, lequel réseau (100) neuronal apprend à identifier un contenu escompté dans la donnée (D) de machine et à reconnaître tout écart à un tel contenu escompté ; pour fournir une norme (WF) de diagnostic et/ou de correction pour une installation (2) électronique d'imagerie médicale, en réaction à une anomalie détectée dans la donnée (D) de machine de cette installation (2) électronique d'imagerie médicale ;
et pour analyser en temps réel dans le centre (3) éloigné d'assistance, la donnée (D) de machine de la norme (WF) de diagnostic et/ou de correction ;
et dans lequel le système (1) de gestion de pannes est réalisé pour permettre un fonctionnement de l'installation (2) électronique d'imagerie médicale depuis le centre (3) éloigné d'assistance, pour effectuer la norme (WF) de diagnostic et/ou de correction.

13. Produit de programme d'ordinateur, comprenant un programme d'ordinateur, qui peut être chargé directement dans une mémoire d'une unité de commande d'un système (1) de gestion de pannes suivant la revendication 12, et qui comprend des éléments de programme pour effectuer des stades du procédé, suivant l'une quelconque des revendications 1 à 11, lorsque le programme d'ordinateur est exécuté par l'unité de commande du système (1) de gestion de pannes.

14. Support déchiffrable par ordinateur pour transporter et/ou mettre en mémoire les parties exécutables du produit de programme d'ordinateur suivant la revendication 13, de manière à ce que celles-ci puissent être déchiffrées par une unité de processeur du système (1) de gestion de pannes.
